# EUROPEAN PATENT APPLICATION

(11) **EP 3 945 022 A1**
(43) Date of publication of application: **02.02.2022**
(21) Application number: 21175173.0
(22) Date of filing: 21.05.2021
(51) Int. Cl.: B64D 13/06, A61L 9/18, F24F 8/192, F24F 8/20

(54) **MULTIFUNCTIONAL COMPOSITE MICROWAVE AIR PURIFIER**

(30) Priority: 31.07.2020 US 202016945273
(71) Applicant: Hamilton Sundstrand Corporation, Charlotte, NC 28217-4578 (US)
(72) Inventor: GANGLOFF, John J., East Hartford, 06118 (US); VAN HASSEL, Bart A., Weatogue, 06089 (US); KHALIL, Yehia F., Glastonbury, 06033 (US); KARRA, Jagadeswara Reddy, Ellington, 06029 (US); SHE, Ying, East Hartford, 06118 (US); DARDAS, Zissis A., Worcester, 01602 (US)
(74) Representative: Dehns

(57) **Abstract**

An air purification device (40) for use in an air management system (10) includes a housing (42) formed from a polymer matrix composite structure and a filter (44) arranged within the housing (42). The filter (44) is formed from a porous composite structure including a plurality of filter fibers (45) such that air is configured to flow through the filter (44). Microbes within the air are configured to contact the plurality of filter fibers (45).

## Description

### STATEMENT OF FEDERAL SUPPORT

This invention was made with Government support under DE-EE0007888 awarded by The Department of Energy. The Government has certain rights in the invention.

### BACKGROUND

Embodiments of the disclosure relate to air purification systems, and more specifically, to an air purification device for use in an air management system of an aircraft.

Pressurized aircraft have integrated air management systems to provide a pressurized environment, fresh air transfer, recycling, heating, and air conditioning to maintain a comfortable safe environment for occupants for extended periods. Air recycling and replacing stale air requires continuous scrubbing for cleanliness to minimize airborne dust, dirt, odors, viruses, spores, and bacteria. This cleaning or scrubbing of the air is typically performed via physical, electrostatic or chemical filtration, such as via a HEPA filter. However, bacteria and dirt can accumulate on the filter, requiring cleaning or replacement of the filters themselves. Further, this purification is arranged at a central portion of the air management system, and as a result, the air could potentially be contaminated as it flows from the filter to the cabin.

### BRIEF DESCRIPTION

According to an embodiment, an air purification device for use in an air management system includes a housing formed from a polymer matrix composite structure and a filter arranged within the housing. The filter is formed from a porous composite structure including a plurality of filter fibers such that air is configured to flow through the filter. Microbes within the air are configured to contact the plurality of filter fibers.

In addition to one or more of the features described above, or as an alternative, in further embodiments the housing polymer matrix composite structure includes a plurality of structural fibers and compliant polymer matrices.

In addition to one or more of the features described above, or as an alternative, in further embodiments one or more dimensions of the housing vary over a length of the housing.

In addition to one or more of the features described above, or as an alternative, in further embodiments the housing and the filter are integrally formed.

In addition to one or more of the features described above, or as an alternative, in further embodiments the plurality of filter fibers are coated with an additive material.

In addition to one or more of the features described above, or as an alternative, in further embodiments the additive material includes an antimicrobial material.

In addition to one or more of the features described above, or as an alternative, in further embodiments comprising an energy source operable to selectively transmit energy to the filter to sterilize the filter.

In addition to one or more of the features described above, or as an alternative, in further embodiments the energy source is operable to emit microwaves.

In addition to one or more of the features described above, or as an alternative, in further embodiments the energy source includes a waveguide and a horn, the horn being arranged in communication with an interior of the filter.

In addition to one or more of the features described above, or as an alternative, in further embodiments comprising an inner conductor arranged within an interior of the filter, and an electromagnetic field is generated between the inner conductor and an inner surface of the housing.

In addition to one or more of the features described above, or as an alternative, in further embodiments a flow path of a cooling fluid extends flows through the inner conductor to remove heat from the filter 44.

In addition to one or more of the features described above, or as an alternative, in further embodiments an air management system of a vehicle having a conditioned area includes at least one duct defining a flow path for delivering air to the conditioned area and at least one air purification device associated with the at least one duct, the at least one air purification device being positioned to provide air purification to a localized portion of the conditioned area.

In addition to one or more of the features described above, or as an alternative, in further embodiments a first air flow from a first source and a second air flow from a second source are provided to the at least one air purification device.

In addition to one or more of the features described above, or as an alternative, in further embodiments the first air flow and the second air flow do not include bleed air drawn from an engine.

In addition to one or more of the features described above, or as an alternative, in further embodiments the conditioned area includes a plurality of sections and the at least one air purification device is positioned to provide the localized air purification between adjacent sections of the plurality of sections.

In addition to one or more of the features described above, or as an alternative, in further embodiments the conditioned area includes a plurality of rows of seats and the at least one air purification device is positioned to provide the localized air purification between adjacent rows of the plurality of rows of seats.

In addition to one or more of the features described above, or as an alternative, in further embodiments the conditioned area includes at least one row of seats and the at least one air purification device is positioned to provide the localized air purification between adjacent seats within the at least one row of seats.

In addition to one or more of the features described above, or as an alternative, in further embodiments the at least one air purification device emits an air curtain.

In addition to one or more of the features described above, or as an alternative, in further embodiments the at least one air purification device forms a portion of the at least one duct.

In addition to one or more of the features described above, or as an alternative, in further embodiments the at least one air purification device further comprises an energy source operable to emit energy into the filter to sterilize the air to be provided to the conditioned area.

### BRIEF DESCRIPTION OF THE DRAWINGS

The following descriptions should not be considered limiting in any way. With reference to the accompanying drawings, like elements are numbered alike:
FIG. 1 is a schematic diagram of an air management system of an aircraft;
FIG. 2 is a front perspective view of an air purification device according to an embodiment;
FIG. 3 is a rear perspective view of an air purification device according to an embodiment;
FIG. 3A is a perspective view of a waveguide including a horn according to an embodiment;
FIG. 4 is a perspective view of an air purification device according to another embodiment;
FIG. 5 is a cross-sectional view of the air purification device according to another embodiment;
FIG. 6 is an end view of a filter including a plurality of fibers according to an embodiment;
FIG. 6A is a schematic diagram of a fiber coated with additive material according to an embodiment; and
FIG. 7 is a schematic diagram of a portion of an interior of a cabin of an aircraft according to an embodiment.

### DETAILED DESCRIPTION

A detailed description of one or more embodiments of the disclosed apparatus and method are presented herein by way of exemplification and not limitation with reference to the Figures.

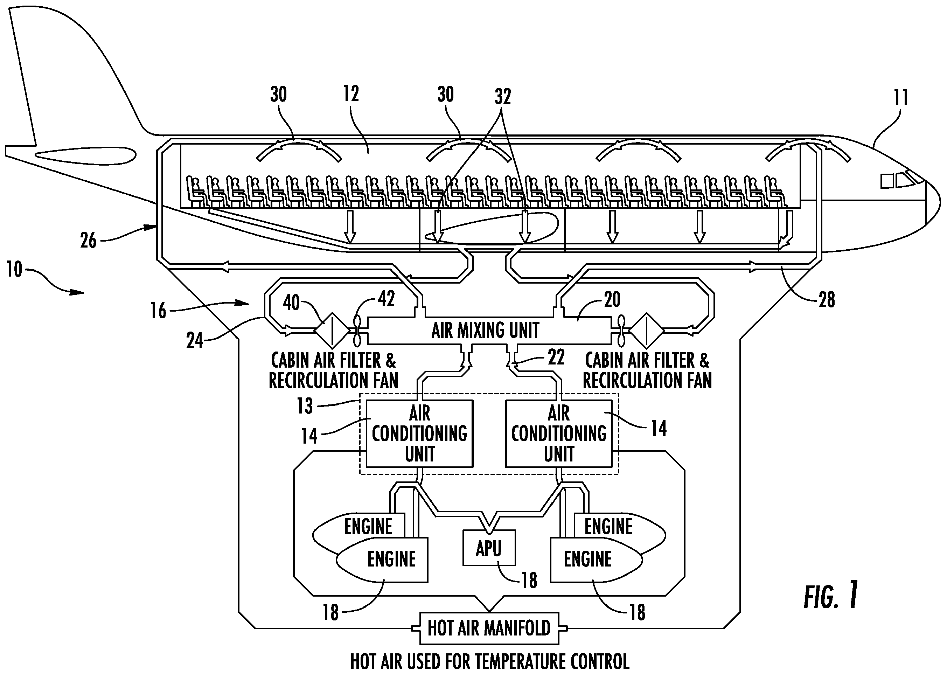

With reference now to FIG. 1, a schematic of an example of an air management system 10 to control the air of a vehicle, such as an aircraft 11 is illustrated. The aircraft 11 includes a pressurized area or cabin 12 that the air management system 10 controls. The cabin 12 may be configured to house people, cargo, and the like therein. The air management system 10 provides conditioned air to, and removes used or contaminated air from, the cabin 12. The air management system 10 includes an environmental control system 13 having at least one air conditioning unit or pack 14, and a cabin air recirculation sub-system 16. While the air management system 10 is illustrated and described herein with reference to an aircraft 11, it should be understood that the systems and techniques discussed herein may be used for a variety of air management systems 10. For example, the cabin 12 may be replaced with any closed volume to be conditioned. As such, systems described herein may be used with ship air management systems, such as submarines and cruise liners for example, personnel carrier air management systems, bus, trolley, train, or subway air management systems, or any other air management system that requires a continual supply of conditioned air.

As shown in the FIG. 1, a medium, such as air for example, is provided from one or more sources 18 to the air management system 10. Examples of suitable sources 18 include but are not limited to an engine of the aircraft 11 and an auxiliary power unit of the aircraft 11. The medium output from these sources 18 is provided to the one or more air conditioning units 14 of the environmental control system 13. Within these air conditioning units 14, the medium is conditioned. This conditioning includes altering one or more of a pressure, temperature, humidity, or flow rate of the medium based on an operating condition of the aircraft. The medium output or discharged from the one or more air conditioning units 14 of the environmental control system 13 may be used maintain a target range of pressures, temperatures, and/or humidity within the cabin 12.

The medium discharged from the air conditioning units 14 is provided to an air mixing unit or mixing manifold 20 via one or more outlet ducts 22. Similarly, at least one duct 24 of the cabin air recirculation sub-system 16 extends from the cabin 12 to the air mixing unit 20 to deliver air exhausted from the cabin 12 to the air mixing unit 20. Within the air mixing unit 20, the cabin recirculating air is mixed with the medium output from the one or more air conditioning units 14 to achieve a mixed medium having one or more desired parameters, such as temperature, pressure, and humidity for example.

In an embodiment, the mixed medium is delivered to the cabin 12 from the air mixing unit 20 via an air distribution system 26 including one or more conduits 28. As shown, the mixed medium may be delivered to the cabin 12 and cockpit via a ventilation system arranged near a ceiling of the cabin 12. In some embodiments, the mixed medium typically circulates from the top of the cabin 12 toward the floor, and is distributed to a plurality of individual vents 30 of the ventilation system spaced laterally between the front and rear of the cabin 12. It should be understood that the air management system 10 illustrated and described herein is intended as an example only, and that any suitable air management system is within the scope of the disclosure. For example, although the air management system 10 illustrated and described herein discloses the use of pressurizes air, also known as "bleed air" drawn from an engine or an auxiliary power unit of the aircraft 11, it should be understood that embodiments of the air management system that are configured to deliver air from another source to the cabin 12, such as fresh air for example, rather than bleed air, are also contemplated herein.

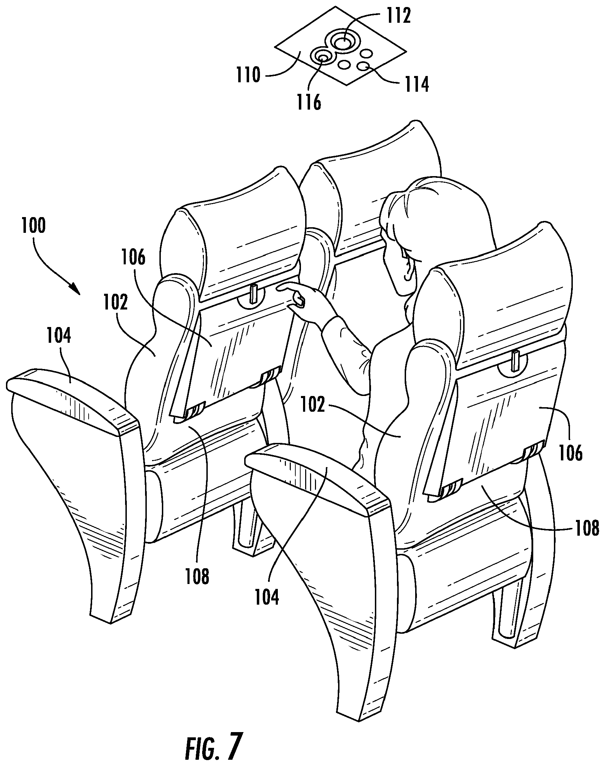

With reference now to FIG. 7, an example of a portion of an interior of a passenger vehicle, such as the cabin 12 of an aircraft for example, is illustrated in more detail. As shown, the cabin 12 includes a plurality of seats 100 which are typically occupied by passengers during flight. In the illustrated, non-limiting embodiment, the passenger seats 100 have a seat back 102 and arm rests 104 disposed on opposing sides of the seat back 102. Further, a tray table 106 movable between a retracted and an extended position is mounted at the back 108 of each passenger seat 22 for use by the passenger seated in the next row. As shown, the plurality of seats 100 are typically grouped into rows. Although two seats are shown in the row of FIG. 7, it should be understood that a row having any suitable number of seats including a single seat, or more than two seats, such as three seats, five seats, or seven seats for example, are also within the scope of the disclosure. Additionally, a passenger service unit 110 is mounted over the passenger seats 100, or at a location generally between adjacent rows of passenger seats 100. The passenger service unit 110 may include at least one light source or reading light 112, an attendant call button 114, and a ventilation supply nozzle 116. In an embodiment, the passenger service unit 110 includes a light source and ventilation supply nozzle 116 for each seat 100 arranged within the row.

One or more air purification devices 40 may be integrated into a portion of the air management system 10, such as the air mixing unit or manifold 20 or a duct portion located directly upstream from the vents 30 operable to expel air into the cabin 12 for example. In an embodiment, air purification devices 40 may be integrated into the air management system 10 to form an air knife or an air curtain between adjacent sections within the cabin 12, between adjacent rows of seats 100, or between seats 100 within a row. In such embodiments, the one or more air purification devices 40 may be used to provide local air purification to passengers of an aircraft. In such embodiments, the one or more air purification devices 40 may be arranged in parallel relative to the flow of the air. Further, in another embodiment, an air flow provided to the cabin 12 may be configured to flow through a plurality of air purification devices 40 arranged in series prior to reaching a vent 30. In such embodiments, a first air purification device along the flow may be configured to purify the air and a second air purification device may be used for filter regeneration. Alternatively, the first and second air purification devices 40 for purifying the air and regenerating the filter may be arranged in parallel.

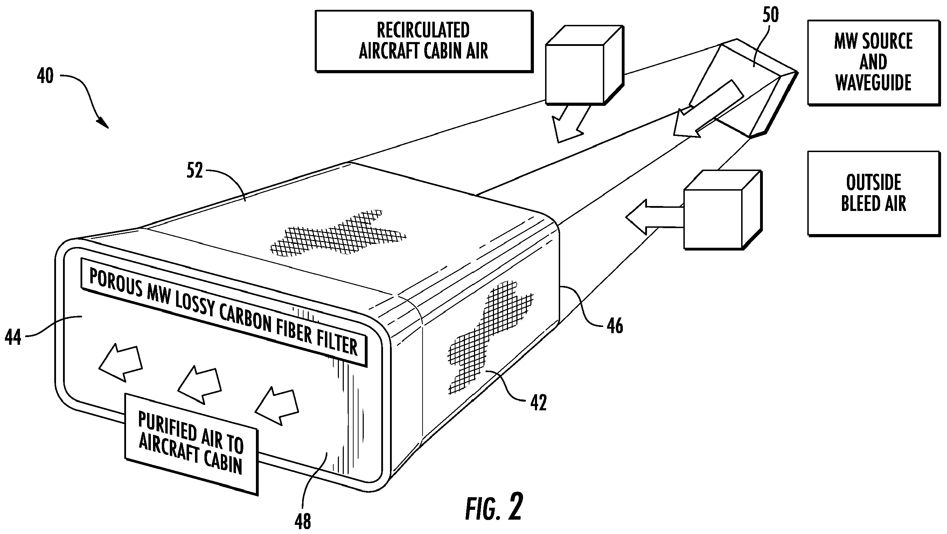

With reference now to FIGS. 2-5, various examples of an air purification device 40 are illustrated in more detail. As shown, the air purification device 40 includes an external housing 42 having a generally hollow interior. In an embodiment, the housing 42 is formed from a lightweight polymer matrix composite (PMC) structure. In an embodiment, the composite structure is built using a plurality of structural fibers formed from any suitable material, including but not limited to carbon, glass, and aramid for example, and compliant polymer matrices. However, it should be understood that in other embodiments, the housing may be formed from other materials, such as from a metallic or plastic structure for example.

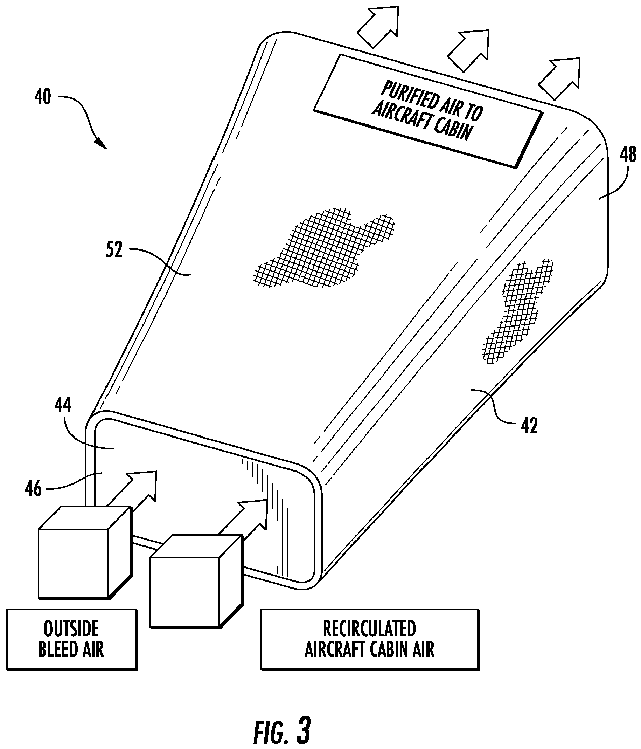

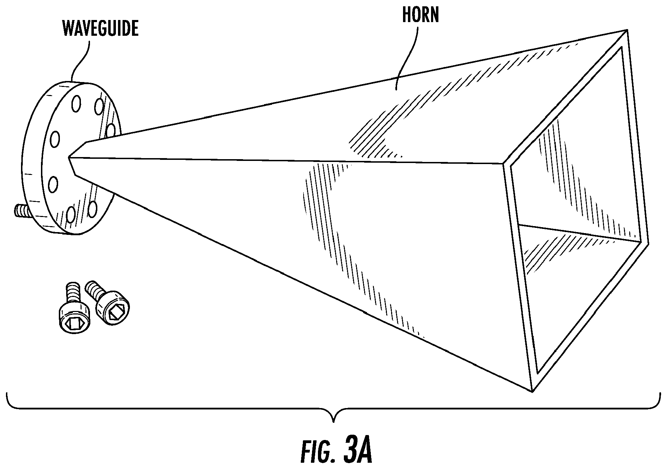

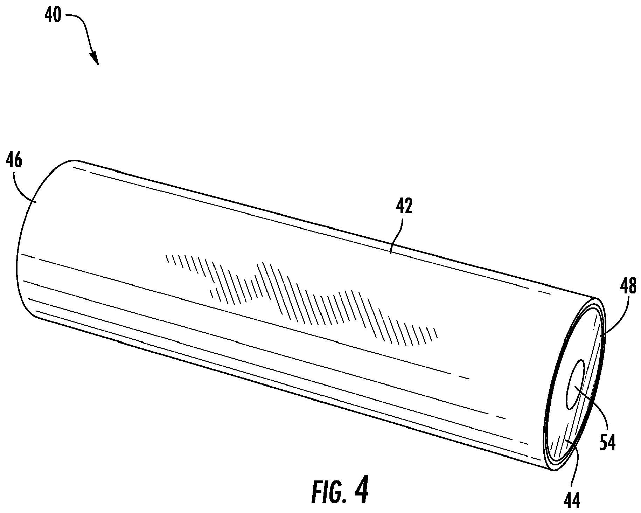

By forming the housing 42 from a composite material, the housing 42 may be fashioned with any geometry. Accordingly, the housing 42 of the air purification device 40 may have a complex geometry, as shown in FIGS. 2 and 3. In such embodiments one or more dimensions of the housing 42 may vary over a length of the housing, such as a curvature along a profile length, or tapering of a cross-sectional area for example. However, it should be understood that any suitable configuration of the housing 42 is within the scope of the disclosure. In other embodiments, the housing 42 of the air purification device 40 may have a simple geometry, as shown in FIGS. 4-5. In such embodiments the housing 42 may have a generally linear configuration and a constant cross-sectional area over its length. In view of the adaptability of the geometry of the housing 42, the exterior profile of the housing 42 of the air purification device 40 may be selected to be complementary to a specific location of the air management system 10, such as a duct or manifold thereof for example.

The construction or composition of the composite structure of the housing 42 may, but need not be, uniform over the entire body of the housing 42. In an embodiment, at least a portion of the composite structure of the housing 42 is non-porous. As used herein, the term "non-porous" is intended to indicate that fluids, such as gas (air) and liquid (water), are not able to circulate there through. The one or more non-porous portions of the housing 42 may be designed to provide structural rigidity to the air purification device 40. Alternatively, or in addition, a portion of the composite structure of the housing 42 may have a porous configuration. In such embodiments, the porous portions of the housing 42 may function as an air purification catalyst support.

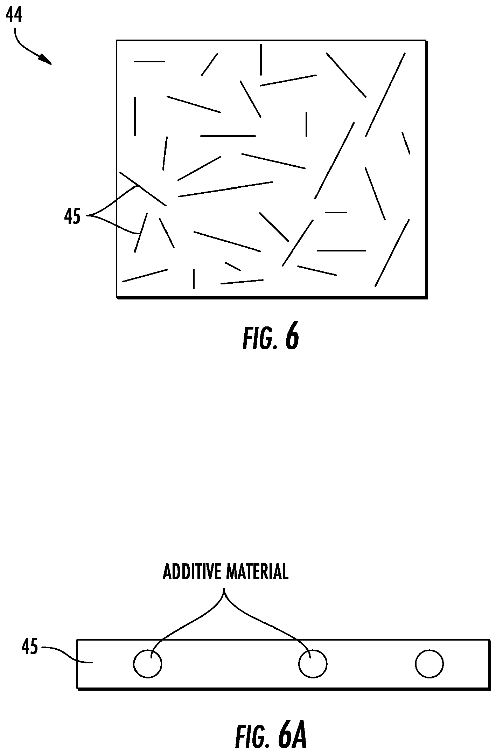

The air purification device 40 additionally includes a filter 44 arranged within the interior of the housing 42. As shown, the filter 44 may extend from a first side or inlet end 46 of the housing 42 to a second, opposite side or outlet end 48 of the housing 42. However, embodiments where the filter 44 has a non-axial configuration are also within the scope of the disclosure. The housing 42 and the filter 44 may be integrally formed with one another. As used herein, integrally formed is intended to indicate embodiments where the housing 42 and the filter 44 are permanently affixed to one another, or embodiments where the housing 42 and filter 44 are formed as a unitary structure. Alternatively, the filter 44 may be a separate component removably connectable to the housing 42 to allow either the housing 42 or the filter 44 to be replaced individually if necessary. In an embodiment, the filter 44 is formed from a porous composite material, for example including porous carbon fibers 45 (see FIG. 6), such that a flow of air is able to travel through the composite material of the filter 44. In another embodiment, the filter 44 may be formed from a material having a metal organic framework (MOF). As used herein, an MOF material is a porous material in which organic ligands and metal ions form a structure having a controllable porosity and pore shape. However, it should be understood that any suitable type of material or fibers 45, including non-porous fibers, may be used to form the filter 44. In addition, the fibers 45 may be continuous, discontinuous, woven, aligned, non-aligned, or any combination thereof.

When installed within an air management system 10 of an aircraft, the air provided to the filter 44 includes one or more of bleed air, fresh air, ram air, cabin recirculation air, or some combination thereof. In an embodiment, the air provided to the filter 44 is a mixture of bleed air and cabin recirculation air. As the air flows through the filter 44, bacteria, viruses, pathogens, volatile organic compounds, and particulate matter within the air flow are trapped by the fibers 45. In an embodiment, at least a portion of the fibers of the filter 44, and in some embodiments the fibers 45 used to form the porous portion of the housing 42, may be coated or doped with an additive material. By using fibers, such as porous carbon fibers within the filter 44, the surface area of the filter 44 that is available to receive a suitable additive, such as via a coating for example, is significantly increased. In an embodiment, the coating material may be capable of sterilizing the microbes removed from the air flow. The sterilization described herein not only includes removal of particulate matter, but also killing or rendering harmless bacteria or airborne viruses within the air purification device 40 and/or an air flow there through.

Examples of suitable additive materials include, but are not limited to an energy absorptive material and air purification catalyst, such as silicon carbide particles, or antimicrobial materials such as silver or copper for example. Any suitable process for coating the fibers 45, such as wet chemistry or adsorption for example, is contemplated herein. Further, the fibers 45 may be coated with one or more additive materials after the formation of the housing 42, or alternatively, during the fiber manufacturing process.

In an embodiment, an energy source 50 is associated with the air purification device 40. In such embodiments, the energy source 50 may be capable of emitting a light or wave having a wavelength suitable to perform purification via germicidal irradiation. In an embodiment, the energy source 50 is capable of emitting microwaves; however other types of energy or waves are also within the scope of the disclosure.

The design or configuration of the housing 42 may be selected based on an electromagnetic field generated by the energy source 50 to maximize microwave losses in the filter 44 and/or enhance air purification performance and quality. Further, the housing 42 may include a material capable of performing electromagnetic interference (EMI) to retain the electromagnetic fields within the structure, and prevent interference with the cables and electronics of the aircraft. Alternatively, or in addition, the housing 42 may include a thermal shielding material to protect the housing 42 from hotspots that could affect the integrity of the structure. This EMI or thermal shielding material may be a separate layer affixed to a surface of the housing 42, or alternatively, may be embedded into the material of the structure.

Alternatively, or in addition, one or more parameters associated with the energy output from the energy source 50 may be selected based on the configuration of the housing 42 and the specific application of the air purification device 40. For example, the microwaves generated by the energy source 50 may be standing waves or travelling waves, and may have a power level of 600W or greater. Further, in an embodiment, the microwaves may have a frequency or 915MHz, or alternatively, a frequency of 2.54 GHz. The parameters described herein are intended as an example only, and it should be understood that any suitable parameter is within the scope of the disclosure.

The energy source 50 may be located adjacent a side of an exterior of the housing 42. In the non-limiting embodiments of FIGS. 2-5, the energy source 50 is positioned adjacent a first side of the housing 42, such as the inlet end 46 of the housing 42 for example. Further, the energy source 50 is arranged in communication with the interior of the air purification device 40 via any suitable mechanism. In the illustrated, non-limiting embodiment, the energy source 50 is formed as one or more horns on a waveguide (FIG. 3A), the horn(s) being connected to an interior of the housing 42, since the energy emitted from the energy source is not capable of penetrating the non-porous body of the housing 42. However, any suitable configuration of the energy source 50 or corresponding component that generates waves that are configured to penetrate into the interior of the filter 44 are contemplated herein. By positioning the energy source 50 adjacent the inlet end 46 or the outlet end 48 of the housing 42, the microwaves emitted by the energy source 50 may, but need not be, generally parallel to a flow of air through the filter 44. It should be understood that any orientation of the waves emitted by the energy source 50 that pass through the filter 44 is within the scope of the disclosure.

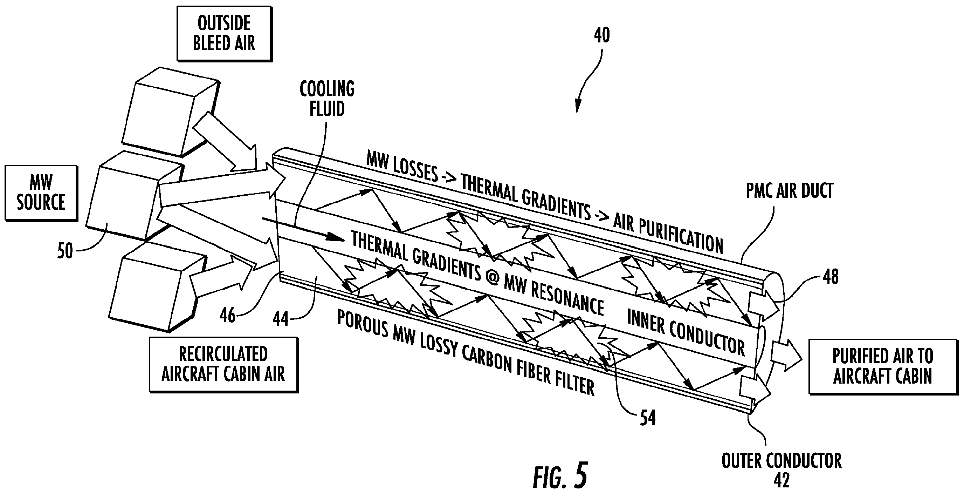

Further, in some embodiments, an inner conductor 54 may be arranged within the interior of the filter 44. In such embodiments, the housing 42 is formed as an outer conductor and an electromagnetic field is generated between the inner conductor 54 and an adjacent surface of the housing 42. The inner conductor 54 may extend over an entire length, or alternatively, only a portion of the length of the housing 42. Further, the inner conductor 54 and the outer conductor may but need not have a coaxial configuration, as shown in FIG. 5. Because microwaves have a limited penetration depth, inclusion of an inner conductor 54 embedded within the filter 44 may be used to evenly dissipate or distribute the microwaves generated by the energy source 50 over a larger volume, such as through the filter 44. Further, in an embodiment, the inner conductor 54 is hollow to allow a cooling fluid to pass there through. Inclusion of a flow of cooling fluid within the inner conductor 54 will facilitate dissipation of absorbed energy from the filter 44, thereby allowing the filter 44 to operate continuously Accordingly, such a configuration can lead to more efficient thermal gradient generation and increase the air purification efficiency of the air purification device 40.

The energy emitted by the energy source 50 may be used not only to purify the air as it passes through the filter 44, but also to regenerate the filter 44 via desorption. As the microwaves penetrate through the filter 44, the microwaves may be selectively absorbed by the particles, such as bacteria or viruses for example. In such embodiments, the microwaves need not heat the material of the filter 44, or the heat may be actively dissipated to maintain the filter 44 at a generally constant temperature. In other embodiments, the microwaves provided to the filter 44 heat the filter 44, including the fibers 45 that are coated with an absorptive material and a catalyst. The use of a microwave typically requires exposure for only a matter of seconds to kill any virus or bacteria present. However, the length of exposure may vary in response to one or more parameters, such as the intensity or strength of the microwave, the volume flow rate of air, and the humidity of the air, for example. As a result, the air purification device 40 does not require regular replacement of the filter 44. Rather, the filter 44 may be regenerated via a regular maintenance operation. As used herein, the term "regenerate" when used in reference to a filter is intended to described purifying or cleaning the filter 44 periodically via exposing the filter 44 to the waves emitted by the energy source. In an embodiment, one or more parameters of the air management system 10, such as an operating pressure or flow temperature for example, may be adjusted when a maintenance operation is performed. Such regular maintenance operations may be scheduled to occur at a fixed time interval, or alternatively, may be performed in response to detection of a parameter of the filter 44, such as a reduced flow rate through the filter 44 for example.

The air purification device 40 may be positioned at any portion of an air management system 10, and therefore may be used to provide localized purification compared to the HEPA filters in existing air management systems. Further, by mounting an energy source 50 adjacent the air purification device 40, the energy source 50 may be used to continuously disinfect the airflow and/or a portion of a filter 44, without exposing aircraft occupants to any harmful effects from exposure to microwaves. Additionally, the air purification device 40 including the energy source 50 could continuously operate when the vehicle is both airborne and grounded without the need for any chemical means of rendering airborne viruses and bacteria harmless.

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

## Claims

1. An air purification device (40) for use in an air management system, comprising:
a housing (42) formed from a polymer matrix composite structure; and
a filter (44) arranged within the housing, the filter being formed from a porous composite structure including a plurality of filter fibers (45) such that air is configured to flow through the filter, wherein microbes within the air are configured to contact the plurality of filter fibers.

2. The air purification device of claim 1, wherein the housing polymer matrix composite structure includes a plurality of structural fibers and compliant polymer matrices.

3. The air purification device of claim 1 or 2, wherein one or more dimensions of the housing (42) vary over a length of the housing.

4. The air purification device of any preceding claim, wherein the housing (42) and the filter (44) are integrally formed.

5. The air purification device of any preceding claim, wherein the plurality of filter fibers (45) are coated with an additive material, and preferably wherein the additive material includes an antimicrobial material.

6. The air purification device of any preceding claim, further comprising an energy source (50) operable to selectively transmit energy to the filter to sterilize the filter.

7. The air purification device of claim 6, wherein the energy source (50) is operable to emit microwaves.

8. The air purification device of claim 6, wherein the energy source (50) includes a waveguide and a horn, the horn being arranged in communication with an interior of the filter.

9. The air purification device of claim 6, 7 or 8, further comprising an inner conductor (54) arranged within an interior of the filter, and an electromagnetic field is generated between the inner conductor and an inner surface of the housing, and preferably wherein a flow path of a cooling fluid extends flows through the inner conductor to remove heat from the filter 44.

10. An air management system (10) of a vehicle having a conditioned area comprising:
at least one duct defining a flow path for delivering air to the conditioned area; and
at least one air purification device (40) as recited in claim 1 associated with the at least one duct, the at least one air purification device being positioned to provide air purification to a localized portion of the conditioned area.

11. The air management system of claim 10, wherein a first air flow from a first source and a second air flow from a second source are provided to the at least one air purification device (40), and preferably wherein the first air flow and the second air flow do not include bleed air drawn from an engine.

12. The air management system of claim 10 or 11, wherein the conditioned area includes a plurality of sections and the at least one air purification device (40) is positioned to provide the localized air purification between adjacent sections of the plurality of sections; or
wherein the conditioned area includes a plurality of rows of seats and the at least one air purification device (40) is positioned to provide the localized air purification between adjacent rows of the plurality of rows of seats; or
wherein the conditioned area includes at least one row of seats and the at least one air purification device (40) is positioned to provide the localized air purification between adjacent seats within the at least one row of seats.

13. The air management system of any of claims 10-12, wherein the at least one air purification device (40) emits an air curtain.

14. The air management system of any of claims 10-13, wherein the at least one air purification device (40) forms a portion of the at least one duct.

15. The air management system of any of claims 10-14, wherein the at least one air purification device (40) further comprises an energy source (50) operable to emit energy into the filter (44) to sterilize the air to be provided to the conditioned area.
